# EUROPEAN PATENT SPECIFICATION

(11) **EP 4 171 402 B1**
(45) Date of publication and mention of the grant of the patent: **09.12.2026**
(21) Application number: 21751831.5
(22) Date of filing: 29.06.2021
(51) Int. Cl.: A61B 17/3211, A61M 16/00

(54) **MEDICAL EQUIPMENT**
MEDIZINISCHE AUSRÜSTUNG
ÉQUIPEMENT MÉDICAL

(30) Priority: 30.06.2020 GB 202009942
(43) Date of publication of application: 03.05.2023
(73) Proprietor: ASMT Group Limited, Reading RG10 0RQ (GB)
(72) Inventor: KARUNAHARAN, Paranjothy, Windlesham Surrey GU20 6JL (GB)
(74) Representative: Slingsby Partners LLP
(86) International application number: PCT/GB2021/051651
(87) International publication number: WO 2022/003346

(56) References cited:
- EP-A1- 3 666 324
- CN-A- 105 963 839
- CN-A- 106 215 294
- SU-A1- 589 988
- US-A- 4 332 245
- US-A- 4 435 853
- US-A1- 2007 088 374
- US-A1- 2011 315 147
- US-B1- 7 267 124

## Description

### Background

This invention relates to a medical equipment, for example equipment for performing procedures such as tracheostomy. The equipment may take the form of a kit.

Tracheostomy is a surgical procedure which involves making an incision, e.g. in the front of a patient's neck, and opening a direct airway through that incision in the trachea. The resulting stoma can serve as a site for a tracheostomy tube to be inserted, allowing the patient to breathe without the use of the nose or mouth.

A currently available tracheostomy tube has several moveable parts. In order to insert the tube, an incision must be made with needles or a scalpel by a skilled medical practitioner. This can be a problem when a tracheostomy is to be performed in an emergency.

Documents CN106215294 A and SU589988 A1 disclose tracheostomy assemblies according to the preamble of claim 1.

Document US 7 267 124 B1 discloses a tracheostomy kit according to the preamble of claim 6.

### Summary

The invention is defined by the subject-matter of independent claims 1 and 6. Preferred embodiments are defined in the dependent claims.

According to one aspect there is provided apparatus for locating an incision on a patient the apparatus comprising: an elongate guide element for guiding a cutting tool to a location at a distal end of the guide element; an optical emitter for emitting an optical beam, the optical emitter being attached to the guide element so that the beam is directed to the distal end of the guide element; and an incision location template slidably attached to the guide element, the incision location template comprising a first feature adapted to be optically correlated with an anatomical feature of the patient and a second feature adapted to be optically aligned with an impingement point of the beam on the patient.

The first feature defined on the template may be defined in such a way that the anatomical feature can be seen through the first feature (e.g. by virtue of the first feature being a void through the template or a transparent region of the template) or around the first feature (e.g. by virtue of the first feature being disposed on a transparent portion of the template). The second feature may be defined so that a beam can shine through it. The first feature may be composed of one or more linear structures. The second feature may be composed of a point feature such as a dot or the intersection of two crossing lines.

According to one aspect there is provided a tracheostomy tube having at a distal end thereof a resilient retention element, the retention element being radially outwardly biased with respect to the tube so as to cause an outer part thereof to expand radially away from the tube when the distal end of the tube extends into a patient's trachea.

The retention element may comprise a wall that is attached to the tube at the distal end of the wall, and whose proximal end is radially expandable about the tube.

According to one aspect there is provided an introducer for a tracheostomy tube, the introducer comprising a tubular body and a two or more wings extending from one end of the body in opposing directions, the tubular body having two or more zones of weakness along its length whereby it can be drawn apart to detach it from a tracheostomy tube passing through the tubular body.

The zones of weakness may be relatively thin regions or perforated regions of the body.

According to one aspect there is provided a bracing element for holding a tracheostomy tube in place on the neck of a patient, the bracing element comprising a locating structure for receiving the tracheostomy tube and one or more flexible bands extending from the locating structure for attachment around the neck of the patient.

According to one aspect there is provided a tracheostomy kit comprising a neck brace securable around a patient's neck, the neck brace defining an aperture arranged to be located at a tracheostomy incision site when the neck brace is secured around the patient's neck, a tracheostomy tube slidably guidable through the aperture, and an introducer guidable through the tracheostomy tube to make an incision at the tracheostomy incision site.

The neck brace may comprise a plate locatable at the front of the patient's neck, and two flexible elements attached laterally to the plate, the flexible elements being attachable to each other at the back of the neck.

The kit may include an incision sleeve within which the tracheostomy tube is enclosed and can be guided into the incision, the incision sleeve having a sharp distal end for widening the incision. The incision sleeve may comprise two halfsleeves, each forming half of a circumference of the sleeve, for ease of removal. The kit further includes a flexible, funnel-shaped drogue, arranged around the tracheostomy tube, which will be deployed inside the trachea when the incision sleeve is split in two and withdrawn. The drogue includes suckers for securing the drogue inside of the trachea.

Each sucker may be a resiliently deformable element of an open and generally conical or hemispherical form which is deformable on pressing against a surface to cause it to adhere to that surface through pressure differential between atmospheric pressure and the pressure between the wall of the sucker and the surface.

The introducer may include a ceramic cutting tip. Alternatively, the introducer may comprise a laser for making the incision or a hybrid.

The introducer may include a manually actuatable part at a proximal end, for advancing the introducer with respect to the tracheostomy tube. A spring may be provided for returning the ceramic cutting edge of the introducer to a retracted state if this version or a hybrid is used instead of the laser.

The tracheostomy tube may be provided with a sensor at a distal location on the tube, for conducting analyses of substances within the patient's trachea, data from the analyses being transferable from the sensor.

The kit may be provided with an ultrasonic attachment for scanning the patient's trachea. A kit as discussed herein may be provided in packaging that takes the form of a sterile barrier enclosing the kit.

Further aspects are set out in the accompanying claims.

### Brief Description of the Drawings

The invention will now be described in more detail, by way of example only, with reference to the accompanying drawings, in which:
Figure 1 is an overhead cross sectional view (as viewed if the patient is supine) of a plate and tube in-situ forming part of a kit according to an embodiment;
Figure 2 is a front view of the plate of Figure 1;
Figure 3 shows the plate of Figure 1 in an oblique view with the tightening clip in both the secured and removed position;
Figure 4 shows the plate of Figure 1 with an incision sleeve and tube in situ;
Figure 5 is a cut-away view of the plate and incision sleeve of Figure 4, showing the tracheostomy tube and the introducer with the spring-loaded action and ceramic blade version;
Figure 6 shows the plate, the incision sleeve, the tracheostomy tube and a drogue;
Figure 7 shows how the tracheostomy tube with a wrap-around drogue is embedded within the incision sleeve;
Figure 8 shows components of the kit in their packaging;
Figure 9 is an exploded view of the packaging of Figure 8;
Figure 10 shows the kit including the neck brace elements;
Figure 11 shows the kit with the neck brace elements fastened together;
Figure 12 shows a ceramic-tipped introducer for the kit in a spring-loaded version;
Figure 13 shows the introducer of Figure 12 with its handle;
Figure 14 is a cut away view of the introducer shown in Figure 12;
Figure 15 shows a hybrid ceramic-tipped/laser introducer;
Figure 16 shows the introducer of Figure 15 with its handle;
Figure 17 is a cut away view of the introducer shown in Figure 15;
Figure 18 shows the plate and the tracheostomy tube with the computer chip removed from the housing;
Figure 19 shows internal details of the tracheostomy tube with the computer chip;
Figure 20 shows an embodiment of the kit with an ultrasound attachment; and
Figure 21 shows the ultrasound attachment removed from the kit.

### Detailed Description

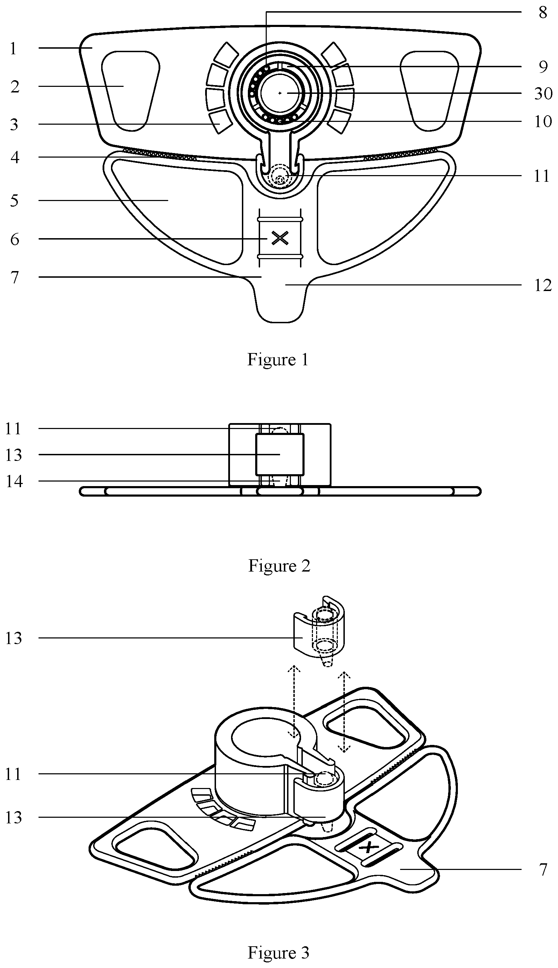

Figures 1, 2 and 3 show a plate for locating the trachea on a patient's neck.

The plate comprises a main upper part 1 and a removable guidance element 7.

The upper part 1 has two apertures 2 at opposite sides for attachment of neck brace components, described below. A central aperture is sized to receive the tracheostomy tube (of different sizes), described below.

The plate has a two-part securing clip 13, apertures 3 in the plate allowing the plate to deform so that the clip can tighten around the tracheostomy tube.

A housing 8 arranged around the central aperture houses wiring for sensors of the kit. There is also a vacuum channel 9 for removing secretions and a channel 10 for transferring the optic fibre cables as a light source.

The detachable guidance element 7 comprises lateral grip portions 5 and has a pulling tab 12 at a frontmost point. A diagram 6 of the trachea is engraved centrally.

A control button 11 for a laser guidance system is housed within the lower part of the clip 13. In a laser-cutting version of the kit, or a hybrid version, this could be housed in the introducer.

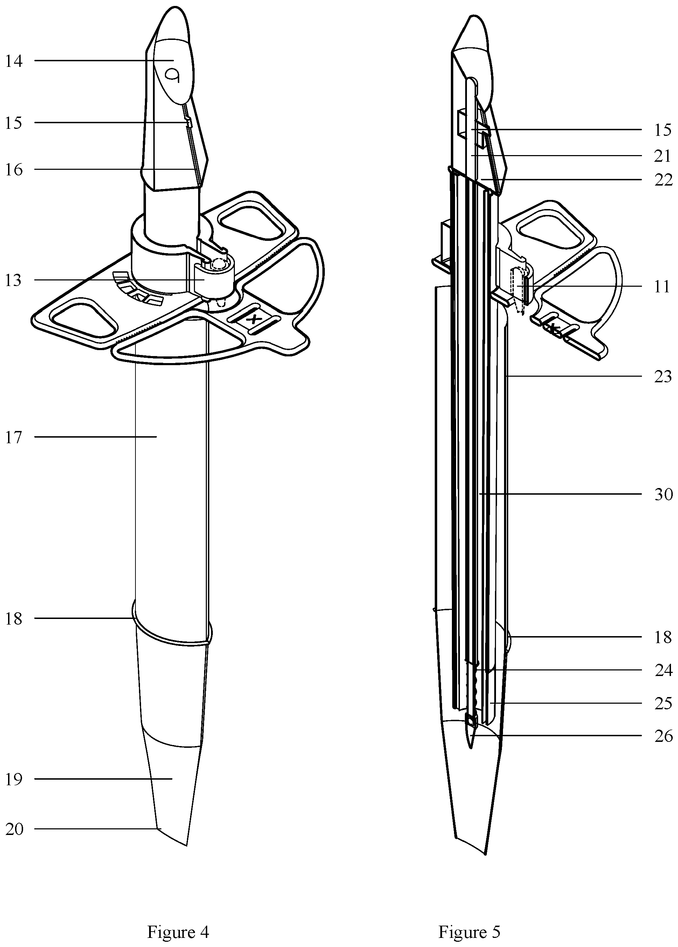

Figures 4 and 5 show the incision sleeve 17 of the tracheostomy kit. The sleeve comprises two longitudinally divided half-sleeves, held together by a serrated edge and support ring 18. The two half-sleeves can be separated from each other like a zip and removed from the support ring to remove the incision sleeve 17. The sleeve has a serrated tapering distal portion 19 with a sharpened end coated in haemostatic material to stop excessive bleeding 20.

Figure 5 shows the tracheostomy tube 30 within the incision sleeve 17. At its distal end the tracheostomy tube has a housing 25 for one or more integrated circuits of the sensor and/or guidance systems.

An introducer 21 is located within the tracheostomy tube. The introducer in this spring-loaded embodiment has a ceramic tip 26 for making the initial incision.

A head 22 at the proximal end of the introducer is located in a handle 14. A button 15 extends through a track 16 in the handle 14 so that, by sliding the button 15, the introducer 21 can be advanced with respect to the tracheostomy tube 30. A spring system 24 retracts the introducer to the position shown when the button 15 is released.

The introducer 21 can also function as a tube housing electronics and the laser guidance or entry system.

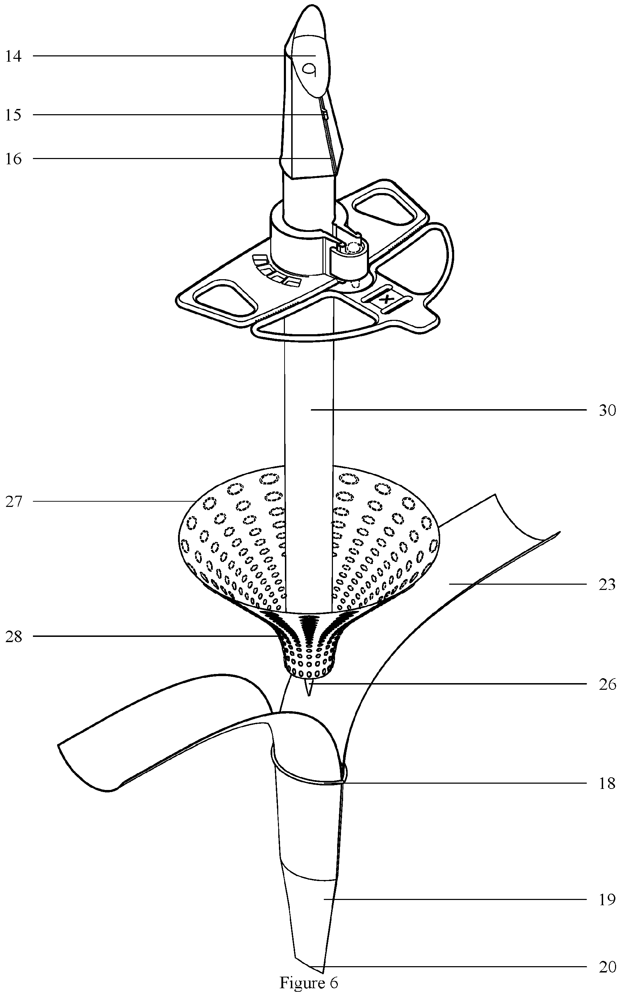

Figure 6 shows a funnel shaped, flexible drogue 27 provided with suction cups 28. The drogue is used to deploy the tracheostomy tube 30 within the trachea and secure it. This is more efficient than the known inflatable cuff or balloon.

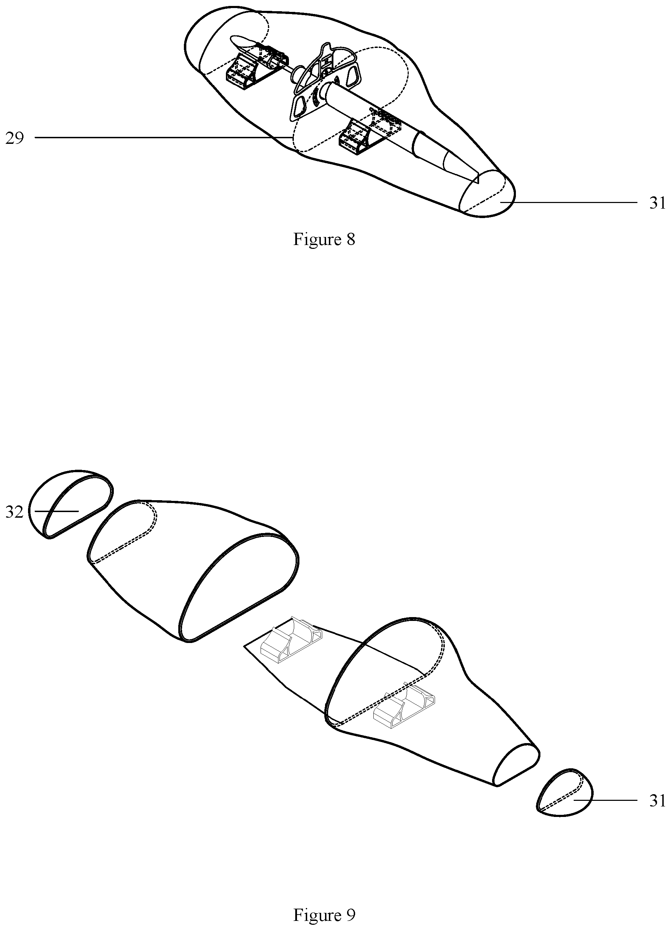

Figures 8 and 9 show packaging for the tracheostomy kit. This comprises a two-part shell 29 with upper and lower removable components 31, 32.

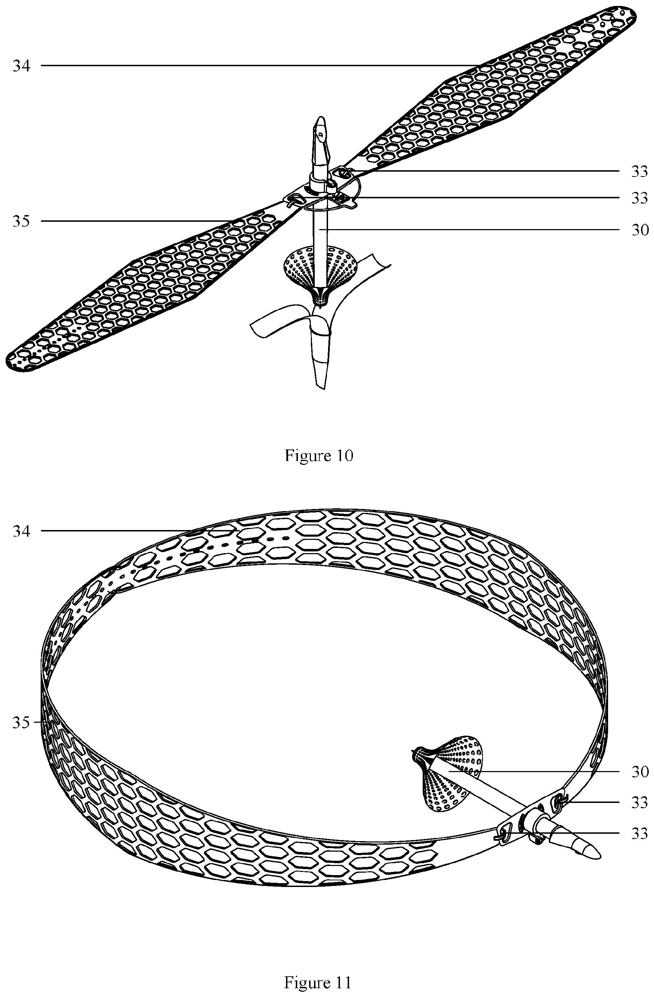

Figures 10 and 11 show the neck brace which holds the plate 1, 7 in place on the patient's neck. The neck brace comprises first and second flexible strap parts 34, 35 which extend around the neck. Male connectors on the first strap part 34 engage in female connectors on the second strap part 35 at the back of the neck.

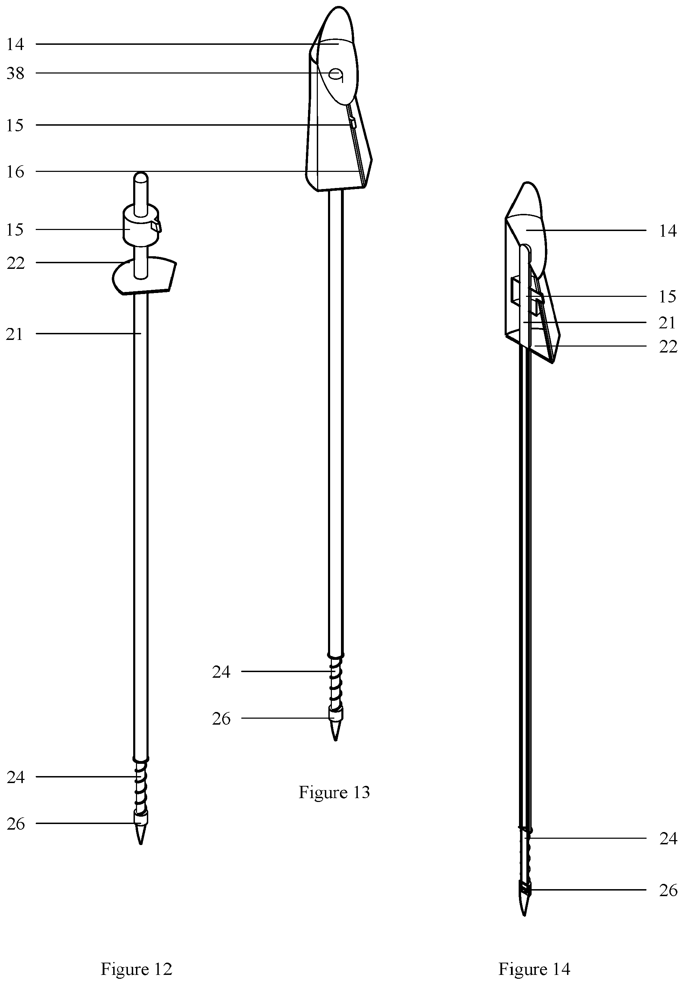

Figure 12 to 14 show details of the introducer 21 with its handle 14. A control button 38 controls the mechanical or laser components of the introducer.

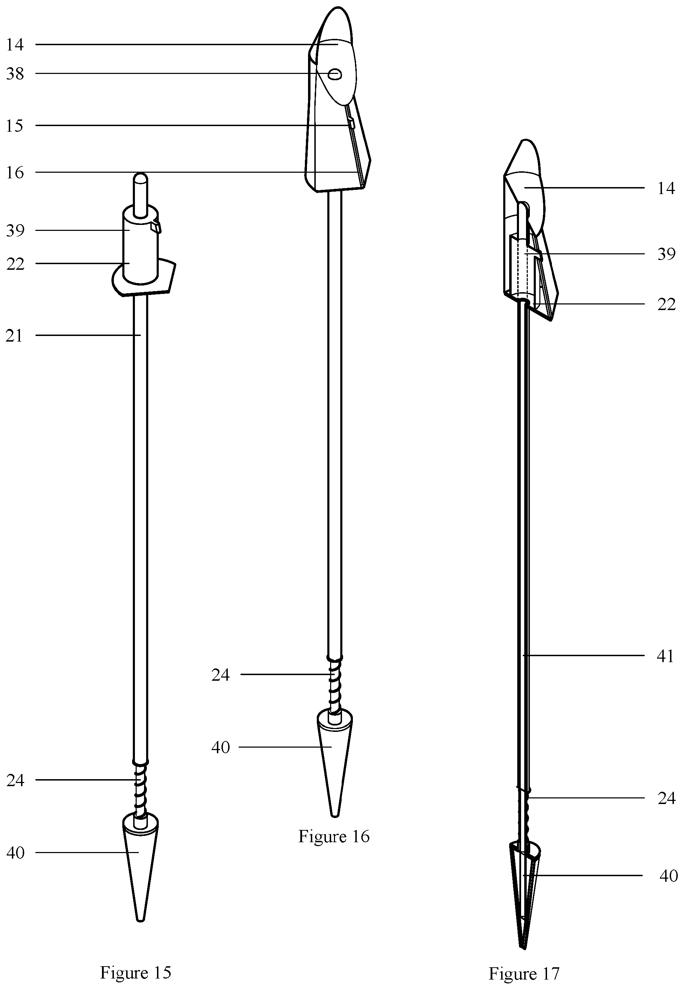

Figures 15 to 17 show a hybrid ceramic-tipped/laser version of the introducer. A housing 39 at the proximal end of the introducer houses controls for the laser/ceramic cutting edge. An external connection can made through the top of the housing 39. A cutting laser is housed within a head 40 at the distal end. The housing 39 communicates with the head 40 via a pressurised tube 41, which can be connected to an external machine.

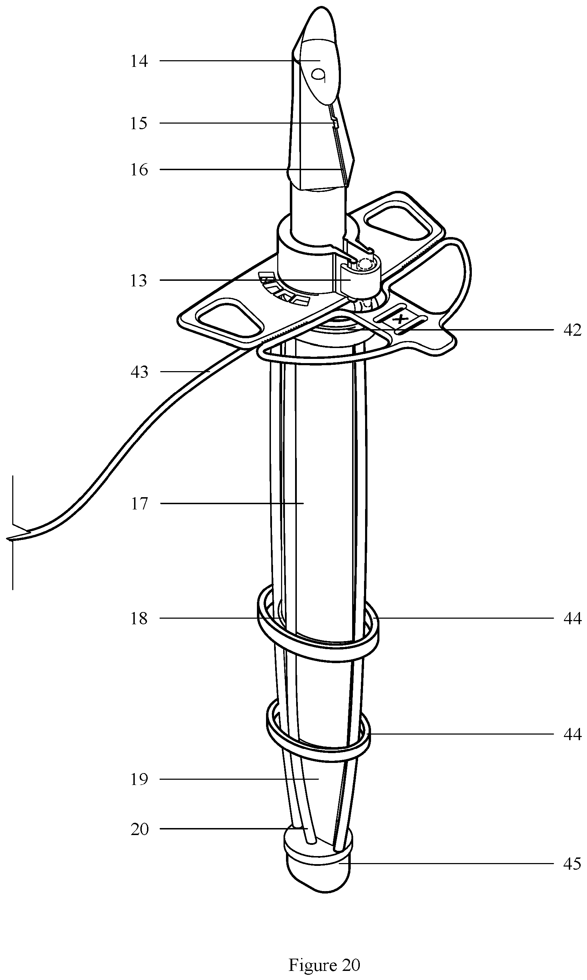

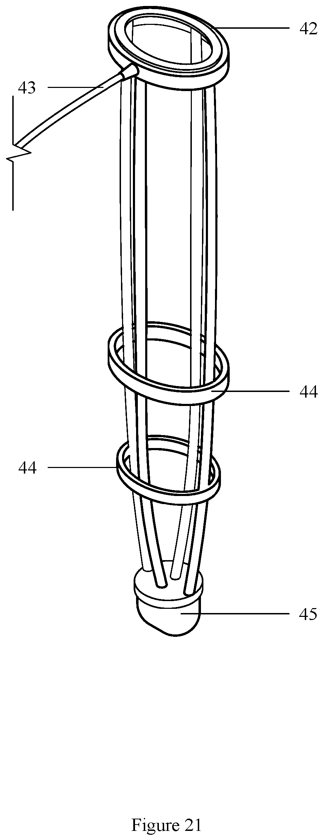

Figures 20 and 21 show an optional ultrasonic attachment which can be attached around the incision sleeve 17. It has an upper housing 42 for wiring and computing components. A cable 43 is provided to connect the attachment to an external device, for example via a USB (universal serial bus) interface. The attachment is secured by means of detachable support clips 44. An ultrasound transducer probe lens 45 is provided at the distal end of the attachment.

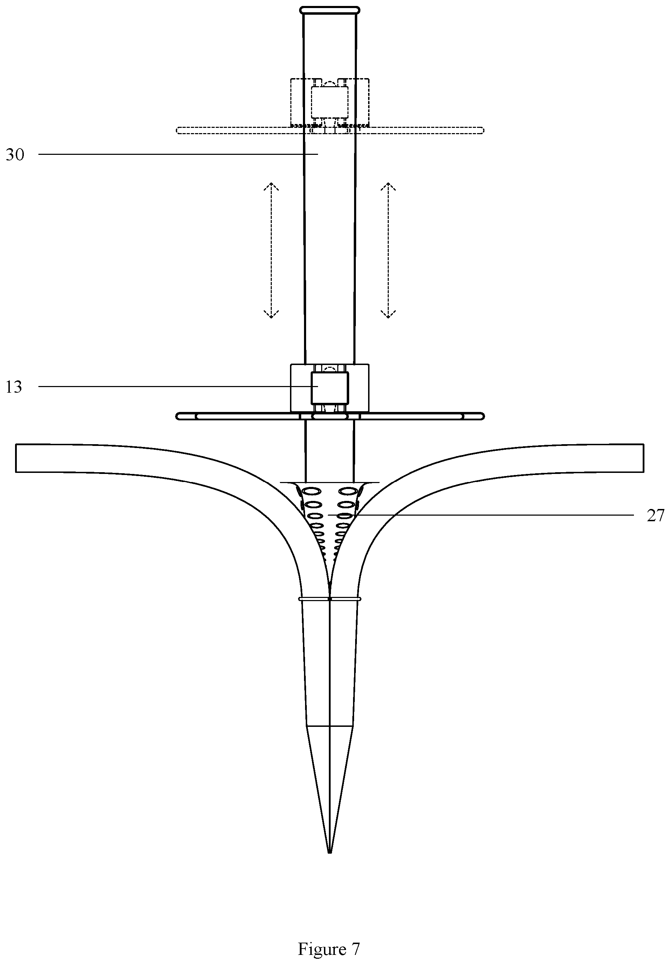

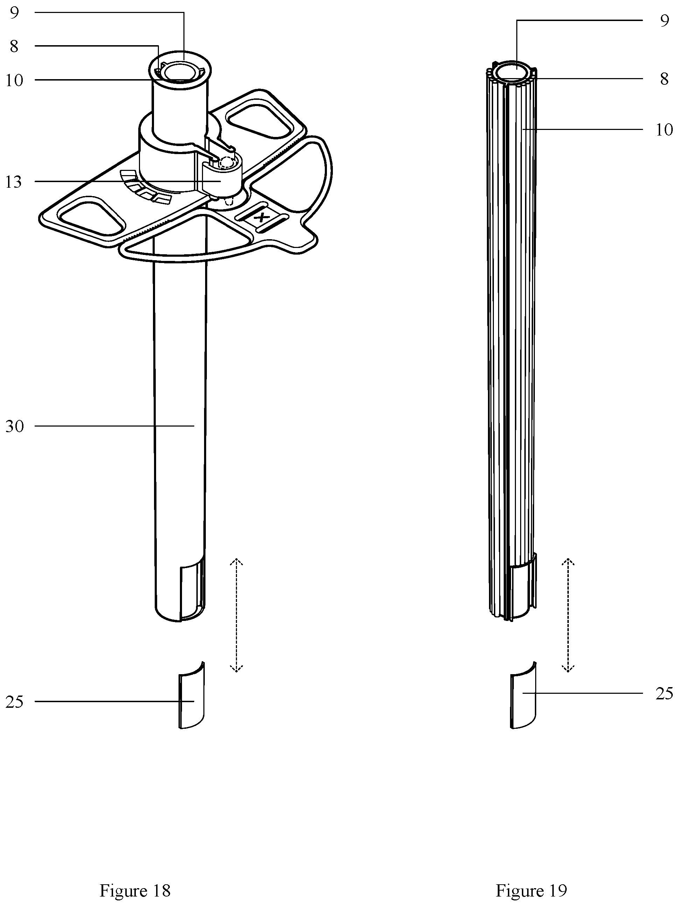

The operation of the equipment shown in figures 1 to 21 will now be described in more detail. The equipment is intended for introducing an artificial airway into a patient's trachea. The airway will be a hollow tube that provides a path between the interior of the trachea and the atmosphere outside the patient's body. Once the airway is introduced, the patient may be able to breathe unaided through the airway. Alternatively, the airway may be connected to a ventilator to assist the patient's breathing.

A first step, which is especially beneficial if the equipment is being used by an unskilled user, is to identify using equipment provided in the kit the location on the patient where the incision is to be made. This may be done in a number of ways, which may each be used individually or with other ways.

A first approach is to use the ultrasonic attachment shown in figure 21. The ultrasonic attachment may be used by itself or may be mounted over the sleeve 30. A user can use the ultrasonic attachment to image a region of the patient where an incision might be made. Data captured by the ultrasonic attachment can be displayed to a user (e.g. on the screen of a coupled smartphone or tablet, or on a dedicated screen). A processor (either in a coupled smartphone tablet or a dedicated processor) may be coupled to a memory storing in a non-transient form code executable by the processor to automatically process ultrasound scan data received from the ultrasonic attachment. It may process the data by comparing it with pre-stored criteria of suitable incision sites to identify when the ultrasonic probe is imaging a suitable incision site. When that is identified, the processor may cause a signal to be issued to a user: for example an audible or visual signal. In one approach, this method may be used to help identify an incision site to a coarse level, e.g. to within 10 to 20mm.

A second approach is to use an optical beam in conjunction with the incision marker 6. The incision marker 6 comprises a cross, whose centre is intended to designate an incision site, and two bars: one on either side of the cross, which are intended to designate cartilage ribs that lie on either side of a suitable incision site. The cross may be replaced by a circle, ring, or other shape that is suitable for designating a point location. The bars may be replaced by lines of circles, ellipses or any other shape that is suitable for designating elongate regions. The bars, cross or other markers are defined by voids so that a user can see through them. Alternatively, the markers could be defined in another way, for example by being deposited or engraved on a transparent plate. This allows a user to sight through the markers at a suitable incision site.

In use, the incision marker, which is carried on a plate 7, is attached to a rigid linear device which is conveniently the tube intended for the tracheostomy or a sleeve for such a tube. The incision marker may be attached to the linear device in such a way that it can slide longitudinally along the linear device. This may be achieved by it being mounted to a clip (e.g. a ring clip or C-clip structure) that runs more than 180 degrees around the linear device. The clip may be a friction fit to the linear device. The incision marker may be hinged to the linear device, e.g. by a living hinge 4. A light source capable of generating a fine optical beam is also attached to the linear device. It may, for example, be a laser or a collimated LED emitter. The light source is arranged to shine a beam parallel with the longitudinal axis of the linear device or directed at the distal tip of the linear device. The light source may, for example, be as shown at 11 and held by clip 30.

The incision marker and the light source are provided with attachments suitable for attaching them to the linear device so that they are held in a configuration suitable for identifying an incision site. This system is used as follows. If they are not already attached, the user attaches the light source and the incision marker to the linear device in a suitable configuration. That will typically be: (a) with the light source arranged to shine along or substantially along the axis of the linear device and (b) with the incision marker offset from the linear device and with its landmarks extending transversely to the linear device. The user sights a suitable incision site through the incision marker. This involves the user visually aligning the bars or other cartilage landmarks with cartilage ribs on the patient's throat. With the user's eye remaining in the same place, the cross or other incision landmark then designates a suitable incision point. The light source operates to shine along the linear device. The beam of the light source is parallel or substantially parallel with the linear device. It may point at the distal tip of the linear device. It may run close to (e.g. within 5mm of the exterior of) the linear device. It may run through the linear device. Thus the light source is configured to shine at a location to which the linear device is aimed. By sliding the incision marker along the linear device and/or by flexing the incision marker relative to the linear device, the user causes the mechanism to adopt a position where (a) the incision marker is aimed at the incision site as described previously and (b) the beam from the light source is visible on the incision site in the centre of the cross or other incision site marker. In this configuration the linear device is aimed at the incision site by virtue of the relationship between the linear device and the light source as discussed above. Now the linear device is in a position to form the incision or to physically guide another component to form the incision. The fact that the incision marker can slide on the linear guide device and/or can be tilted relative to the guide device about a revolute joint 4 whose axis is orthogonal to the axis of the linear guide device can assist a user in aligning the optical beam and the incision landmarks on the incision marker.

If the linear device is equipped with a cutting tip, e.g. a sharp physical tip or an optical cutting tip, e.g. of the types described above, then it may be advanced by the user to and through the incision site to form an incision into the patient. If the linear device is a guide for another cutting device, e.g. if it is a tube or rail through or along which a cutting device can be introduced then it may be held in the position that has been adopted by using the technique described above and then the cutting device advanced through or along it. The linear device may have extensions that deviate from its linear extent. The linear device may alternatively be curved or od another shape. The optical emitter could then emit a beam that is directed to a cutting site on the device or to an exit point of a guide on the device to which the device can guide a cutting tool.

Once the incision has been made, or the incision site marked, or the guide fixed in place relative to the incision site, the locating equipment shown in figure 3 can be removed from the guide tube.

Once the linear device is in position it may be held there by coupling wings 34 around the neck of the patient. In one convenient method, the linear device is a guide. The guide is positioned as described above. Then the guide is optionally held in place by the wings 34. Then a cutter such as shown in any of figures 12 to 17 is introduced through the guide to form the incision.

The guide may be equipped with a separator comprising 18, 19, 20, 23 as shown in figure 6 located on the exterior distal end of the guide. The separator may be perforate or perforable by the cutter at its distal end. The cutter may be extended through the separator to form the incision. Once the incision is made, the separator may be advanced into the incision. The separator may comprise a port 18 and two or more wings 23 which splay outwards proximally of the port. Thus the separator can stabilise an opening at the incision. The guide or another element suitable for use as a tracheostomy tube may then be advanced through the separator.

The guide or other tracheostomy tube may be provided with a drogue or flange 27 (see figure 6) at its distal end. The flange may be capable of adopting a first configuration in which it is flush with the exterior of the tracheostomy tube and a second configuration (see figure 6) in which its periphery is expanded to a location spaced from the exterior of the tube. The flange may be elastically biased to the second configuration. The flange may be formed of an elastically deformable sheet which is configured to naturally adopt the second configuration. With such a flange, the tracheostomy tube can be advanced through the incision and/or port. As this is done the flange adopts the first configuration, so it does not obstruct insertion of the tube. When the distal end of the tube reaches the patient's trachea the flange springs to the second configuration. In the second configuration the flange can bear on the interior of the patient's trachea to resist withdrawal of the tracheostomy tube. This can reduce the chance of the tube being withdrawn unintentionally.

In the second configuration the flange may adopt a conical or part-conical shape, being wider proximally as shown in figure 6. This can help to resist withdrawal. Since the flange expands without intervention from the user when it reaches the trachea it can be more suitable for use by an unskilled operator than, e.g. an inflatable balloon. The flange can also serve to help stabilise the trachea.

The flange can also serve to collect fluid in the trachea. That fluid can be removed by suction, e.g. along one or more ancillary tubes running alongside or embedded in the wall of the tracheostomy tube. Fluids removed in this way can be analysed to help assess the state of the patient. Fluids can be introduced through one or more such ancillary tubes. An endoscope can be introduced through such an ancillary tube. The flange/drogue may also serve to catches foreign matter falling down the trachea and/or to hold fluids that can be sampled, e.g. for diagnostics.

When it is desired to remove the tracheostomy tube, the tube can be pulled to cause the conical flange to invert through pressure against the interior of the trachea.

To assist the user, the tracheostomy tube may have a marker on its exterior which indicates a suitable depth of insertion into a patient.

Optionally the separator may be removed once the tracheostomy tube is properly inserted. A tracheostomy tube may be a tube suitable for serving as an airway in a patient who has received a tracheostomy.

Compared to a conventional tracheostomy tube, the tube described above may have fewer moveable parts and may house a twin target system, i.e. both mechanical and laser guided, for accurate entry. The laser guide means can also be located at the neck or further down at the tip of the introducer where it can also be used for incision.

The kit can optionally be formed without metal parts (except for the spring system). Thus it may not require needles or a scalpel to insert as it uses the ceramic tipped introducer. The introducer may be coated with haemostatic material to inhibit excessive bleeding.

The neck brace secures the apparatus to the neck. It is unlike current systems which use cloth. It and the tracheostomy tube can be provided in large, regular, small and paediatric sizes for ease of use.

The tracheostomy tube described herein can be an electronic "smart" tube with a chip in place for onboard sensors to analyse gases and chemicals with the data being transferable to the outside. It will have an arc shaped fine fibre-optic light source embedded. The tube will also house an automated self cleaning system for secretions which is currently done manually. The tube is designed to be placed in-situ for an extended period of time, for example three or more years as opposed to 28 days with some existing systems. Inert lightweight material may be used. It may be coated in a material for haemostasis to stop excess bleeding due to the incision. Once in situ in patients, it is automated and connected to the ventilator, light sources and a vacuum source. Unlike the tube typically inserted at present by ear nose & throat surgeons, the present device is designed to be intuitive to use and to be inserted by non-clinicians including the public.

The apparatus described above has been described in the context of performing tracheostomy. The apparatus may be used for performing other procedures that involve the making of an incision in a suitable place on a patient and/or the introduction of a tube through such an incision. Examples may include the introduction of a venous catheter or a cannula. The incision markers could be adapted to designate landmarks appropriate to other incision sites. The patient could be a human or animal patient. The user of the apparatus could be the patient themselves.

One or more items of the apparatus described above may be provided together as a kit. They may be provided together in an airtight container such as a sealed plastics bag. The apparatus of the kit may be sterile when in the container. Some elements of the apparatus may be provided externally to the container. Items of the apparatus may be provided to the end user in assembled form, partially assembled form or fully disassembled form. The end user may assemble one or more of the parts together to cause the apparatus to adopt a usable configuration as described above.

The phrase "configured to" or "adapted to" followed by a term defining a condition or function is used herein to indicate that the object of the phrase is in a state in which it has that condition, or is able to perform that function, without modification or further configuration/adaptation. The word "for" followed by a term defining a condition or function is used herein to indicate that the object of the phrase is suitable for adopting that condition or performing that function.

## Claims

1. A tracheostomy assembly comprising a tracheostomy tube (30) having at a distal end thereof a resilient retention element (27) the retention element being radially outwardly biased with respect to the tube so as to cause an outer part thereof to expand radially away from the tube when the distal end of the tube extends into a patient's trachea, **characterised in that** the retention element is provided with suckers (28) on its outer face for attachment to the interior of the patient's trachea.

2. The tracheostomy tube as claimed in claim 1, wherein the retention element, when expanded, has the form of a conical wall extending about the tube, the radially larger end of the conical wall being more proximal relative to the tube than the radially smaller end of the conical wall.

3. The tracheostomy tube as claimed in claim 2, wherein the wall is perforate.

4. The tracheostomy tube (30) as claimed in any of claims 1 to 3, the tracheostomy tube having a wall defining a principal interior lumen through which a patient may breathe when the tube is inserted into the patient's trachea and one or more subsidiary interior lumens (9, 10) extending through the wall from one end of the tube to the other whereby fluid communication can be made to the interior of the patient's trachea when the tube is inserted therein.

5. The tracheostomy tube as claimed in claim 4, defining multiple such subsidiary lumens, each subsidiary lumen being separated from the others by material of the wall.

6. A tracheostomy kit comprising a neck brace (1, 34, 35) securable around a patient's neck, the neck brace defining an aperture arranged to be located at a tracheostomy incision site when the neck brace is secured around the patient's neck, a tracheostomy tube (30) slidably guidable through the aperture, and an introducer (21) guidable through the tracheostomy tube to make an incision at the tracheostomy incision site, **characterised in that** the kit includes a resilient retention element (27) which is radially outwardly biased, formed as a flexible, funnel-shaped flange (27), and is arranged around the tracheostomy tube, for retaining the tracheostomy tube in the trachea and wherein the funnel-shaped flange includes suckers (28) on its outer face for securing to the inside of the trachea.

7. The tracheostomy kit according to claim 6, wherein the neck brace comprises a plate (1) locatable at the front of the patient's neck, and two flexible elements (34, 35) attached laterally to the plate, the flexible elements being attachable to each other at the back of the neck.

8. The tracheostomy kit according to claim 6 or 7, including an incision sleeve (17) through which the tracheostomy tube can be guided into the incision.

9. The tracheostomy kit according to claim 8 wherein the incision sleeve has a sharp distal end (26) for widening the incision.

10. The tracheostomy kit according to claim 8 or 9, wherein the incision sleeve comprises two half-sleeves (23), each forming half of a circumference of the sleeve.

11. The tracheostomy kit according to any of claims 6 to 10, wherein the introducer includes a ceramic cutting tip (26).

12. The tracheostomy kit according to any of claims 6 to 11, wherein the introducer comprises a laser (40) for making the incision.

13. The tracheostomy kit according to any of claims 6 to 12, wherein the introducer includes a manually actuatable part (15) at a proximal end, for advancing the introducer with respect to the tracheostomy tube.

14. The tracheostomy kit according to claim 13, including a spring (24) for returning the introducer to a retracted state.

15. The tracheostomy kit according to any of claims 6 to 14, comprising an ultrasonic attachment (42, 44, 45) for scanning the trachea.

## Patentansprüche

1. Tracheostomieanordnung, die einen Tracheostomietubus (30) umfasst, der an einem distalen Ende davon ein elastisches Halteelement (27) aufweist, wobei das Halteelement in Bezug auf den Tubus radial nach außen vorgespannt ist, um zu bewirken, dass sich ein äußerer Teil davon radial von dem Tubus weg erweitert, wenn sich das distale Ende des Tubus in eine Trachea eines Patienten hinein erstreckt, **dadurch gekennzeichnet, dass** das Halteelement an seiner Außenfläche mit Saugnäpfen (28) zur Anbringung an dem Innenbereich der Trachea des Patienten versehen ist.

2. Tracheostomietubus nach Anspruch 1, wobei das Halteelement, wenn es erweitert ist, die Form einer konischen Wand aufweist, die sich um den Tubus erstreckt, wobei das radial größere Ende der konischen Wand in Bezug auf den Tubus proximaler ist als das radial kleinere Ende der konischen Wand.

3. Tracheostomietubus nach Anspruch 2, wobei die Wand perforiert ist.

4. Tracheostomietubus (30) nach einem der Ansprüche 1 bis 3, wobei der Tracheostomietubus eine Wand aufweist, die ein Hauptinnenlumen, durch das ein Patient atmen kann, wenn der Tubus in die Trachea des Patienten eingeführt ist, und ein oder mehrere Nebeninnenlumen (9, 10), die sich von einem Ende des Tubus zu dem anderen durch die Wand erstrecken, definiert, wobei eine Fluidverbindung zu dem Innenbereich der Trachea des Patienten hergestellt werden kann, wenn der Tubus darin eingeführt ist.

5. Tracheostomietubus nach Anspruch 4, der mehrere solcher Nebenlumen definiert, wobei jedes Nebenlumen durch Material der Wand von den anderen getrennt ist.

6. Tracheostomiekit, das eine Zervikalstütze (1, 34, 35), die um den Hals eines Patienten befestigbar ist, wobei die Zervikalstütze eine Öffnung definiert, die so angeordnet ist, dass sie sich an einer Tracheostomie-Inzisionsstelle befindet, wenn die Zervikalstütze um den Hals des Patienten befestigt ist, einen Tracheostomietubus (30), der gleitend durch die Öffnung führbar ist, und eine Einführeinrichtung (21), die durch den Tracheostomietubus führbar ist, um eine Inzision an der Tracheostomie-Inzisionsstelle vorzunehmen, umfasst, **dadurch gekennzeichnet, dass** das Kit ein elastisches Halteelement (27) umfasst, das nach außen vorgespannt ist, als ein flexibler, trichterförmiger Flansch (27) ausgebildet ist und zum Halten des Tracheostomietubus in der Trachea um den Tracheostomietubus angeordnet ist, und wobei der trichterförmige Flansch Saugnäpfe (28) auf seiner Außenfläche zur Befestigung an der Innenseite der Trachea umfasst.

7. Tracheostomiekit nach Anspruch 6, wobei die Zervikalstütze eine Platte (1), die an der Vorderseite des Halses des Patienten angeordnet werden kann, und zwei flexible Elemente (34, 35), die seitlich an der Platte angebracht sind, umfasst, wobei die flexiblen Elemente aneinander an der Rückseite des Halses angebracht werden können.

8. Tracheostomiekit nach Anspruch 6 oder 7, das eine Inzisionshülse (17) umfasst, durch die der Tracheostomietubus in die Inzision geführt werden kann.

9. Tracheostomiekit nach Anspruch 8, wobei die Inzisionshülse ein scharfes distales Ende (26) zum Aufweiten der Inzision aufweist.

10. Tracheostomiekit nach Anspruch 8 oder 9, wobei die Inzisionshülse zwei Halbhülsen (23) umfasst, die jeweils eine Hälfte eines Umfangs der Hülse bilden.

11. Tracheostomiekit nach einem der Ansprüche 6 bis 10, wobei die Einführeinrichtung eine keramische Schneidspitze (26) umfasst.

12. Tracheostomiekit nach einem der Ansprüche 6 bis 11, wobei die Einführeinrichtung einen Laser (40) zum Vornehmen der Inzision umfasst.

13. Tracheostomiekit nach einem der Ansprüche 6 bis 12, wobei die Einführeinrichtung einen manuell betätigbaren Teil (15) an einem proximalen Ende zum Vorschieben der Einführeinrichtung in Bezug auf den Tracheostomietubus umfasst.

14. Tracheostomiekit nach Anspruch 13, das eine Feder (24) zum Zurückführen der Einführeinrichtung in einen zurückgezogenen Zustand umfasst.

15. Tracheostomiekit nach einem der Ansprüche 6 bis 14, das einen Ultraschall-Aufsatz (42, 44, 45) zum Abtasten der Trachea umfasst.

## Revendications

1. Ensemble de trachéotomie comprenant un tube de trachéotomie (30) ayant à une extrémité distale de celui-ci un élément de retenue élastique (27), l'élément de retenue étant sollicité radialement vers l'extérieur par rapport au tube de manière à amener une partie extérieure de celui-ci à se dilater radialement en s'éloignant du tube lorsque l'extrémité distale du tube s'étend jusque dans la trachée d'un patient, **caractérisé en ce que** l'élément de retenue est muni de ventouses (28) sur sa face extérieure pour fixation à l'intérieur de la trachée du patient.

2. Tube de trachéotomie selon la revendication 1, dans lequel l'élément de retenue, lorsqu'il est dilaté, a la forme d'une paroi conique s'étendant autour du tube, l'extrémité radialement plus grande de la paroi conique étant plus proximale par rapport au tube que l'extrémité radialement plus petite de la paroi conique.

3. Tube de trachéotomie selon la revendication 2, dans lequel la paroi est perforée.

4. Tube de trachéotomie (30) selon l'une quelconque des revendications 1 à 3, le tube de trachéotomie ayant une paroi définissant une lumière intérieure principale à travers laquelle un patient peut respirer lorsque le tube est inséré dans la trachée du patient et une ou plusieurs lumières intérieures subsidiaires (9, 10) s'étendant à travers la paroi depuis une extrémité du tube jusqu'à l'autre, moyennant quoi une communication fluidique peut être établie à l'intérieur de la trachée du patient lorsque le tube y est inséré.

5. Tube de trachéotomie selon la revendication 4, définissant de multiples lumières subsidiaires de ce type, chaque lumière subsidiaire étant séparée des autres par le matériau de la paroi.

6. Kit de trachéotomie comprenant un collier cervical (1, 34, 35) pouvant être fixé autour du cou d'un patient, le collier cervical définissant un orifice agencé pour être situé au niveau d'un site d'incision de trachéotomie lorsque le collier cervical est fixé autour du cou du patient, un tube de trachéotomie (30) pouvant être guidé de manière coulissante à travers l'orifice, et un introducteur (21) pouvant être guidé à travers le tube de trachéotomie pour réaliser une incision au niveau du site d'incision de trachéotomie, **caractérisé en ce que** le kit comporte un élément de retenue élastique (27) qui est sollicité radialement vers l'extérieur, formé comme une bride flexible en entonnoir (27), et est agencé autour du tube de trachéotomie, pour la retenue du tube de trachéotomie dans la trachée et dans lequel la bride en entonnoir comporte des ventouses (28) sur sa face extérieure pour une fixation à l'intérieur de la trachée.

7. Kit de trachéotomie selon la revendication 6, dans lequel le collier cervical comprend une plaque (1) pouvant être placée à l'avant du cou du patient, et deux éléments flexibles (34, 35) attachés latéralement à la plaque, les éléments flexibles pouvant être attachés l'un à l'autre à l'arrière du cou.

8. Kit de trachéotomie selon la revendication 6 ou 7, comprenant un manchon d'incision (17) à travers lequel le tube de trachéotomie peut être guidé jusque dans l'incision.

9. Kit de trachéotomie selon la revendication 8, dans lequel le manchon d'incision présente une extrémité distale pointue (26) destinée à élargir l'incision.

10. Kit de trachéotomie selon la revendication 8 ou 9, dans lequel le manchon d'incision comprend deux demi-manchons (23), chacun formant la moitié d'une circonférence du manchon.

11. Kit de trachéotomie selon l'une quelconque des revendications 6 à 10, dans lequel l'introducteur comporte une pointe coupante en céramique (26).

12. Kit de trachéotomie selon l'une quelconque des revendications 6 à 11, dans lequel l'introducteur comprend un laser (40) destiné à réaliser l'incision.

13. Kit de trachéotomie selon l'une quelconque des revendications 6 à 12, dans lequel l'introducteur comporte une partie pouvant être actionnée manuellement (15) au niveau d'une extrémité proximale, pour faire avancer l'introducteur par rapport au tube de trachéotomie.

14. Kit de trachéotomie selon la revendication 13, comportant un ressort (24) destiné à ramener l'introducteur dans un état rétracté.

15. Kit de trachéotomie selon l'une quelconque des revendications 6 à 14, comprenant un accessoire à ultrasons (42, 44, 45) destiné à balayer la trachée.
